(19) Europäisches Patentamt / European Patent Office / Office européen des brevets

(11) **EP 3 285 645 B1**

(12) **EUROPEAN PATENT SPECIFICATION**

(45) Date of publication and mention of the grant of the patent:
**12.08.2020 Bulletin 2020/33**

(21) Application number: **16717650.2**

(22) Date of filing: **21.04.2016**

(51) Int Cl.:
*A61B 5/03* (2006.01)     *A61B 5/00* (2006.01)
*A61M 16/08* (2006.01)     *A61M 16/14* (2006.01)
*A61M 11/00* (2006.01)     *A61M 15/00* (2006.01)
*A61B 5/087* (2006.01)     *A61M 15/08* (2006.01)
*A61M 16/00* (2006.01)     *A61M 16/04* (2006.01)

(86) International application number:
**PCT/EP2016/058953**

(87) International publication number:
**WO 2016/170087 (27.10.2016 Gazette 2016/43)**

(54) **SYSTEM FOR EFFECTIVE BREATH-SYNCHRONIZED DELIVERY OF MEDICAMENT TO THE LUNGS**

SYSTEM ZUR EFFEKTIVEN UND ATEMSYNCHRONISIERTEN ABGABE EINES MEDIKAMENTS IN DIE LUNGEN

SYSTÈME POUR UNE ADMINISTRATION EFFICACE, SYNCHRONISÉE AVEC LA RESPIRATION, D'UN MÉDICAMENT AUX POUMONS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **22.04.2015 EP 15164749**
**15.06.2015 EP 15172198**

(43) Date of publication of application:
**28.02.2018 Bulletin 2018/09**

(73) Proprietor: **Chiesi Farmaceutici S.p.A.**
**43122 Parma (IT)**

(72) Inventors:
• **DELLACA', Raffaele**
  **43122 Parma (IT)**
• **MILESI, Ilaria**
  **43122 Parma (IT)**

(74) Representative: **PGA S.p.A.**
**Via Mascheroni, 31**
**20145 Milano (IT)**

(56) References cited:
**WO-A1-2013/160129**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

**Field of technology**

The present invention relates to the field of retropharyngeal instillation of medicament and particularly to a system for the administration of a pulmonary surfactant by atomization with a breath/synchronized delivery.

**Background of the invention**

Preterm infants are prone to develop IRDS (Infant Respiratory Distress Syndrome) because of generalized lung immaturity. Nowadays, clinical management of preterm RDS infants mostly relies on 1) providing respiratory support and 2) administering exogenous pulmonary surfactant. The current most widely accepted approach for providing respiratory support to newborns is focused on avoiding invasive mechanical ventilation and intubation in favour of non-invasive respiratory support approaches such as nasal continuous positive airway pressure (CPAP), nasal intermittent positive pressure ventilation (NIPPV) or high flow nasal cannula (HFNC) whenever mechanical ventilation is not strictly necessary. Even if these approaches showed good results, they are not directly addressing surfactant deficiency and a significant number of newborns still requires exogenous surfactant therapy. Pulmonary surfactant administration is currently performed by bolus administration, as this is the only clinically proven effective administration approach. Unfortunately, bolus administration requires intubation, a complex and invasive procedure that might be associated to several side-effects. Moreover, bolus administration is often associated to hemodynamic and systemic fluctuations, due to the amount of liquid administered in the lungs and due to the sudden following reduction of lung resistance, that, in turn, are considered further potential risks for the babies. Because of these implications, a great effort has been dedicated in finding alternative ways for administration of pulmonary surfactants. In particular, the administration by nebulization has been extensively studied. In more details, commercial nebulizer were inserted along the ventilator circuit. So far, the results of these trials are inconclusive, showing very poor surfactant deposition into the lungs.

In turn, poor deposition could be due to one or more of the following occurrences: 1) a significant amount of surfactant deposits along the ventilator connections, 2) if the patient is under CPAP, the surfactant that is not inspired deposits in the upper airways and is then swallowed by the newborn instead of achieving the lungs, and 3) if the nebulizer is not synchronized with the neonate's breath, the surfactant nebulized during expiration is exhaled.

A possible alternative approach that could overcome these issues was proposed by Wagner et al. (Wagner MH, Amthauer H, Sonntag J, Drenk F, Eichstädt HW, Obladen M "Endotracheal surfactant atomization: an alternative to bolus instillation?" Crit Care Med. 2000; 28(7):2540). They used a modified tracheal tube with an atomizer inserted at the tip of the tube, which produced relatively big particles compared to typical nebulization (-100 μm) only during inspiration (manually synchronized by an operator). Despite big technological issues and despite the need for intubation, this approach showed to be effective and was further developed by Chiesi and Polytechnic of di Milano (see Patent Application WO2013/160129). The resulting implemented embodiment provides an improvement aiming at overcoming the limitations of previous approaches. In fact this device allows: 1) to deliver surfactant in the newborn's pharyngeal region during spontaneous breathing and 2) to maximize the quantity of surfactant delivered by synchronizing the atomization with the inspiratory breathing phase. The device disclosed in WO2013/160129 is schematically shown in Figure 1: it includes a small and flexible atomizing catheter that can be inserted in the pharyngeal region of the patient connected to an infusion pump that delivers the liquid medicament (for example surfactant) and source of compressed gas. The atomizing catheter includes a first channel where the liquid medicament flows to be conveyed to the patient's pharyngeal region and a second channel that conveys a pressurized flow of gas at the tip of the catheter. The pump moves the column of liquid medicament towards the tip of the catheter so that, when the medicament and the pressurized gas meet in the pharyngeal cavity, the liquid is broken into small particles causing atomization of the medicament and the delivery of the drug into the patient's lungs.

The system further includes a pressure sensor along the surfactant line for measuring a value indicative of the pressure in the patient pharyngeal cavity, such value being used to determine whether the patient is in an inspiration or in an expiration phase and to activate the pump only during the inspiratory phase. The pressure measured along the surfactant line will be the sum of two contributions: 1) the drop of pressure due to the medicament flow through the catheter and 2) the pressure swings due to the breathing activity.

Since the medicament lumen is very thin and the medicament could be viscous, the flow resistance associated to this channel can be very high. Moreover, as the liquid medicament has to be loaded into the system and, during this procedure, it is very likely that small air bubbles appears into the medicament line and these bubbles, being compressible, behave like hydraulic compliances. The coupling of high resistance and compliances leads to potentially high time constants that have two detrimental impacts on the operation of the system:

1) long rising time in the pressure of the medicament when the infusion pump is activated, leading to long delays from when the volumetric pump starts to when the surfactant flows at the tip of the catheter.

2) it prevents a prompt detection of the breathing phase needed to synchronize the delivery of surfactant in phase with breathing as the breathing signal can be delayed and masked by the motor activation.

In patent application WO2013/160129 the delivery of the medicament and the sensing of the pressure has been done with a single catheter lumen, but this is only one of the possible implementations. For instance, in co-pending patent application PCT/EP2014/072278 filed by the same Applicants, Chiesi and Polytechnic of Milano and not published yet, an alternative embodiment is disclosed embodiment with a separate channel dedicated to the sensing of the breath.

By adding a dedicated sensing line, it has been possible to overcome the issue due to the detection of the breathing, since the sensing line can be designed without the strict constrains of the atomizing catheter, but, on the other hand, issue related to delay in the delivery still remains.

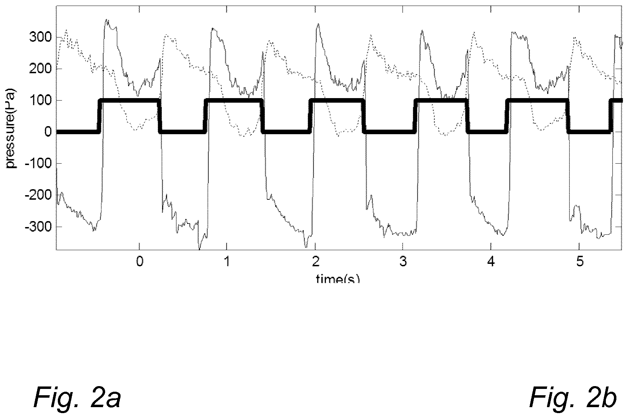

If special care is used to remove all air bubbles, the pressure signal recorded on the medicament line allows the detection of the respiratory phase. In Figure 2 it is possible to appreciate the reproducibility between the pressure measured along the surfactant line and the one recorded in the pharynx when a careful priming is performed and almost no bubbles are inserted into the circuit. The diagram on Figure 2 shows the comparison of the following values:

- pressure measured along the medicament (surfactant) line (solid line);
- pharyngeal pressure (dashed line);
- infusion pump motor activation signal (thick line).

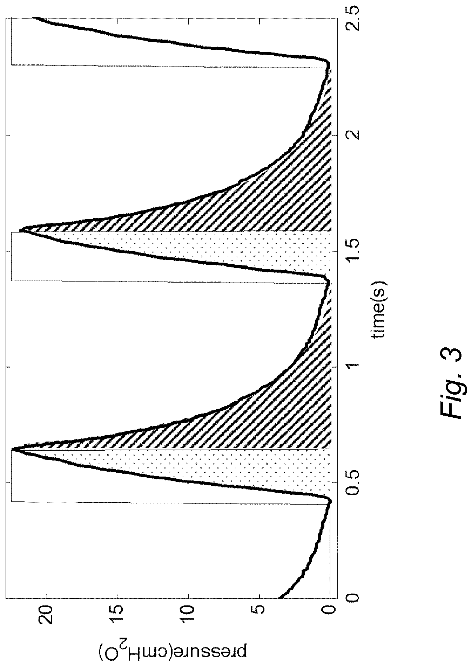

The diagram of Figure 2 shows the behaviour of the pressure measured along the medicament line during synchronized medicament delivery in case a very small amount of bubbles (e.g. 0.01ml in total) has been inserted during the priming procedure. These conditions are achievable by special care in removing gas bubbles from the medicament circuit, but require a skilled operator to perform the priming and it requires long time. Therefore, conditions of Figure 2, are unfortunately unlikely to happen. In case of bad priming of the circuit, the time constant of the system increases and in extreme conditions with big amount of bubbles, it could lead to dramatic impacts on the response of the system as shown in Figure 3.

The consequence of prolonged rising time is mainly that the time in which the medicament starts flowing in the atomizing tip is "delayed" compared to the time in which the infusion pump is turned on, compromising the possibility to administer the medicament only during the inspiratory phase and, therefore, reducing the total amount of medicament potentially deliverable to the patient's lung during each inspiration, leading to longer time for completing the treatment and waste of medicament. Moreover, when the pump is turned off, because of the long-time constant the flow does not immediately stop and part of the medicament is wasted as delivered during expiration.

In Figure 3, the thin line represents the motor activation and the expected pressure, the thick line represents the actual pressure (proportional to the flow) sensed on the surfactant line. The dotted area represents the amount of medicament that is properly administered during the inspiration, while the dashed area represents the medicament being wasted as delivered during the expiration of the patient.

Another side effect of the increased time constant is related to the detection of the breathing activity. If the rising time is too long, the breathing activity is totally masked by the activation of the motor, which has a biggest amplitude (it is equal to surfactant flow time catheter hydraulic resistance) compared to the breathing activity of the baby, preventing from detecting respiratory phase on the surfactant line.

Possible options to reduce the time constant of the system are:

1) Reduce the hydraulic resistance of the surfactant lumen. This can be done but if the surfactant lumen is too big, the airflow needed to atomize will be very high, because the surface contact between surfactant and compressed air is small compared to the cross section of the surfactant lumen. High airflow, such as higher than 1.5 litres per minute (LPM) are not compatible with the system, therefore this solution cannot be implemented.

2) Reduce the compliance of the system. This can be obtained by using rigid components, such as glass syringes and optimizing the mechanical components of the infusion pumps and above all, reducing the amount of bubbles in the surfactant with a careful priming, which is the preeminent cause for compliance. Unfortunately, this procedure is very time consuming and it is difficult to completely de-bubbling the system. Moreover the result of the priming procedure is quite unpredictable, making the working condition of the system difficult to define.

Both proposed adjustment options described above are rather heavy and time consuming and cannot completely solve the problem.

For all these reasons, an improved method and system for administering the exogenous pulmonary surfactant which is capable of compensating the delay in the delivery caused by the mechanical and hydraulic characteristics of the system would be greatly appreciated. Moreover, the possibility of implementing this method without requiring major changes in the mechanical and electronic components is another desirable feature.

**Objects of the invention**

It is an object of the present invention to overcome at least some of the problems associated with the prior art.

**Summary of the invention**

The present invention provides a system as set out in the accompanying claims.

According to one aspect of the present invention, we provide a system for delivering a liquid pulmonary surfactant to spontaneously breathing patients according to claim 1.

In a preferred embodiment adapting the flow produced by the first pump means includes increasing the initial flow of the first pump means until the pressure measured by the pressure detecting means reaches a predetermined value.

The predetermined sets of coefficients can be stored in a lookup table accessible by the microprocessor.

In an embodiment of the present invention the breathing detecting means includes a pressure sensor, for measuring a value indicative of the pressure in the patient pharyngeal cavity, such value being used to determine whether the patient is in an inspiration or in an expiration phase. In a possible optional embodiment the determination of whether the patient is in an inspiration or in an expiration phase is done by detecting the start of the inspiration phase and calculating the end of the inspiration phase according to predetermined values indicative of the duration of the inspiration phase.

According to an embodiment of the present invention, the pressure sensor coincides with the pressure detecting means connected to the first channel. Alternatively the breathing detecting means can use a separate channel to detect the inspiration and expiration phases.

In any case the pressure detecting means and the breathing detecting means can be connected and provide feedback to the microprocessor which will calculate the proper corrective actions to counterbalance the hydraulic impedance according to measured values received from the pressure detecting means and from the breathing detecting means..

In a preferred embodiment the catheter includes at least one dedicated gas channel adapted to convey in the patient's pharyngeal region a pressurized flow of gas, the system further comprising: gas pump means connected to a first end of the gas channel, adapted to create the flow of pressurized gas; so that when the column of liquid medicament and the pressurized gas meet in the pharyngeal cavity, the liquid column is broken into a plurality of particles causing the atomized medicament to be delivered into the patient's lungs.

Preferably, the aerosol medicament comprises an exogenous pulmonary surfactant, e.g. selected from the group consisting of modified natural pulmonary surfactants (e.g. poractant alfa), artificial surfactants, and reconstituted surfactants.

Also, in a preferred embodiment, the pressurized gas includes air or oxygen. A still further aspect of the present disclosure provides a computer program for controlling the above described method.

The system of the invention could be utilized for the prevention and/or treatment of the respiratory distress syndrome (RDS) of the neonate (nRDS) and of the adult (ARDS) as well as for the prevention and/or treatment of any disease related to a surfactant-deficiency or dysfunction such as meconium aspiration syndrome, pulmonary infection (e.g. pneumonia), direct lung injury and bronchopulmonary dysplasia.

Therefore, a further aspect of the present disclosure is directed to the use of a pulmonary surfactant administered by means of the above described system for the prevention and/or treatment of the aforementioned disease. The system of the present invention provides an efficient delivery of the aerosol medicament, controlling the behavior of the infusion pump to make the rising and falling time faster even though the intrinsic time constant of the system is long. Additionally, in an embodiment of the present invention, at the same time the information about the breathing activity contained either directly on the surfactant line or stored in the controller action can be used to extrapolate the breathing pattern. The system of the present invention provides several advantages including the use of components which are already familiar to the hospital personnel, e.g. catheters and disposable pressure sensors; all the part in contact with the pulmonary surfactant and the patient are low cost and disposable, granting for hygienically and safe treatments, which is particularly important when the patient is a pre-term neonate.

**Brief description of the drawings**

Reference will now be made, by way of example, to the accompanying drawings, in which:

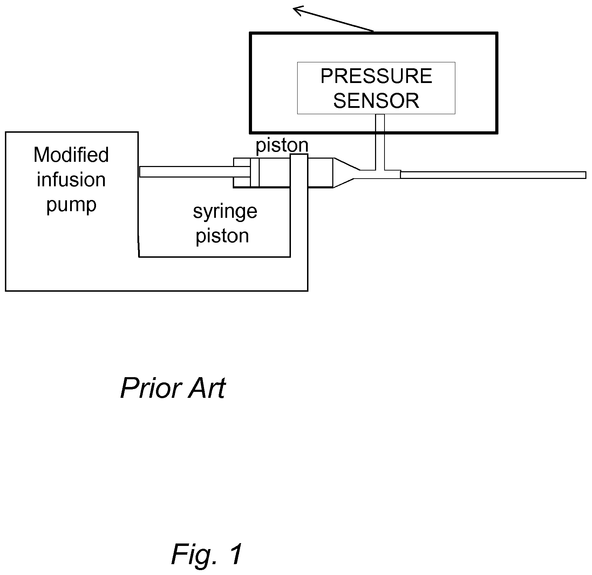

Figure 1 is a schematic diagram of one of the possible systems of the prior art;
Figures 2-3 show the behavior of the pressure measured along the medicament line during synchronized medicament delivery in ideal conditions (Fig.2) and in real world cases (Fig. 3) according to the prior art system of Fig.1;

Figure 4 schematically represents the mathematical description of a system according to an embodiment of the present invention, while Figure 5 is a block diagram of the same;

Figure 6 shows a schematic mathematical representation of a system according to a preferred embodiment of the present invention;

Figure 7 is a schematic block diagram of a system according to a preferred embodiment of the present invention;

Figure 8 shows a schematic representation of a system according to a preferred embodiment of the present invention;

Figure 9 schematically represents a comparison between the behaviors of the Open loop control system vs Closed loop control system;

Figure 10 is a picture representing an implementation of a system according to an embodiment of the present invention;

Figure 11 represents the result obtained with a system according to an embodiment of the present invention;

Figures 12-24 are schematic representations of systems, block diagrams, results and performances of further embodiments of the present invention.

## Definitions

The tem "liquid medicament" encompasses any medicament wherein the active ingredient is dissolved or suspended in the liquid medium, preferably suspended. The terms "neonates" and "newborns" are used as synonymous to identify very young patients, including pre-term babies having a gestational age of 24 to 36 weeks, more particularly between 26 and 32 weeks.

With the term "pulmonary surfactant" it is meant an exogenous pulmonary surfactant administered to the lungs that could belong to one of the following classes:

i) "modified natural" pulmonary surfactants which are lipid extracts of minced mammalian lung or lung lavage. These preparations have variable amounts of SP-B and SP-C proteins and, depending on the method of extraction, may contain non-pulmonary surfactant lipids, proteins or other components. Some of the modified natural pulmonary surfactants present on the market, like Survanta™ are spiked with synthetic components such as tripalmitin, dipalmitoylphosphatidylcholine and palmitic acid.

ii) "artificial" pulmonary surfactants which are simply mixtures of synthetic compounds, primarily phospholipids and other lipids that are formulated to mimic the lipid composition and behavior of natural pulmonary surfactant. They are devoid of pulmonary surfactant proteins;

iii) "reconstituted" pulmonary surfactants which are artificial pulmonary surfactants to which have been added pulmonary surfactant proteins/peptides isolated from animals or proteins/peptides manufactured through recombinant technology such as those described in WO 95/32992 or synthetic pulmonary surfactant protein analogues such as those described in WO 89/06657, WO 92/22315, and WO 00/47623.

The term "non-invasive ventilation (NIV) procedure" defines a ventilation modality that supports breathing without the need for intubation.

## Detailed description of preferred embodiments

With reference to the accompanying figures an implementation of the system according to a preferred embodiment of the present invention is illustrated. In the example here discussed we addressed the problem of delivering the right amount of atomized medicament to a patient: in particular we administrated a pulmonary surfactant to a patient population e.g. a preterm neonate. The utilized pulmonary surfactant is poractant alfa, formulated as 80 mg/ml aqueous suspension and commercially available as Curosurf® from Chiesi Farmaceutici SpA.

However, any pulmonary surfactant currently in use, or hereafter developed for use in respiratory distress system and other pulmonary conditions could be suitable for use in the present invention. These include modified natural, artificial and reconstituted pulmonary surfactants (PS).

Current modified natural pulmonary surfactants include, but are not limited to, bovine lipid pulmonary surfactant (BLES™, BLES Biochemicals, Inc. London, Ont), calfactant (Infasurf™, Forest Pharmaceuticals, St. Louis, Mo.), bovactant (Alveofact™, Thomae, Germany), bovine pulmonary surfactant (Pulmonary surfactant TA™, Tokyo Tanabe, Japan), poractant alfa (Curosurf®, Chiesi Farmaceutici SpA, Parma, Italy), and beractant (Survanta™, Abbott Laboratories, Inc., Abbott Park, III.)

Examples of artificial surfactants include, but are not limited to, pumactant (Alec™, Britannia Pharmaceuticals, UK), and colfosceril palmitate (Exosurf™, GlaxoSmithKline, plc, Middlesex).

Examples of reconstituted surfactants include, but are not limited to, lucinactant (Surfaxin™, Discovery Laboratories, Inc., Warrington, Pa.) and the product having the composition disclosed in Table 2 of Example 2 of WO2010/139442. Preferably, the pulmonary surfactant is a modified natural surfactant or a reconstituted surfactant. More preferably the pulmonary surfactant is poractant alfa (Curosurf®). In another preferred embodiment, the reconstituted surfactant has composition disclosed in WO2010/139442 (see Table 2 of Example 2 of WO2010/139442).

The dose of the pulmonary surfactant to be administered varies with the size and age of the patient, as well as with the severity of the patient's condition. Those of skill in the relevant art will be readily able to determine these factors and to adjust the dosage accordingly.

Other active ingredients could advantageously be comprised in the medicament according to the invention including small chemical entities, macromolecules such as proteins, peptides, oligopeptides, polypeptides, polyamino acids nucleic acid, polynucleotides, oligo-nucleotides and high molecular weight polysaccharides, and mesenchimal stem cells derived from any tissue, in particular from a neonate tissue. In a particular example, small chemical entities include those currently used for the prevention and/or treatment of neonatal respiratory diseases, for example inhaled corticosteroids such as beclometasone dipropionate and budesonide.

The method according to an example of the present disclosure exploit mathematical concepts which are common to various field of technology (e.g. hydraulic, electronic, automation and controlling theory); in the following paragraph we are providing a brief description of the basic concepts.

## DESCRIPTION OF THE SYSTEM

The description of the invention will be accompanied by mathematical formulation and modeling which should help in understanding the problems solved by the present invention

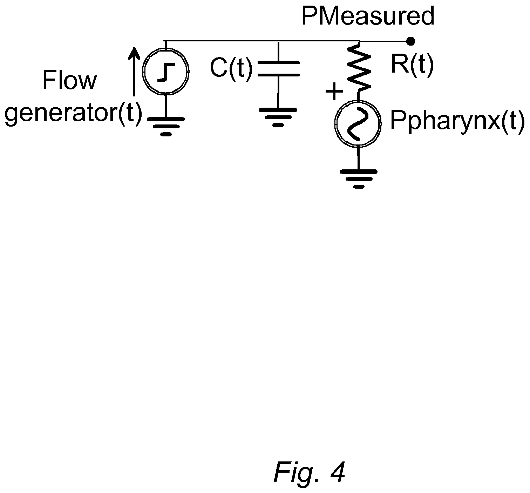

The comprehensive delivery system for the medicament and the pharynx environment can be modelled in a very simplified, although consistent way, as described in Figure 4

Where:

C(t) = compliances of the system, mainly due to bubbles. It may be time dependent because it could change according to the amount of foam and bubbles.

R(t)= resistance of the system, mainly due to the catheter. It could be time depedent because partial occlusions or kinking of the catheter.

Flow generator(t) = this is the action of the volumetric pump.

Ppharynx(t)= pressure at the pharynx due to the breathing activity and ventilatory support.

Pmeasured(t) = pressure measured along the surfactant line, which is due to the activation of infusion pump and breathing activity of the patient.

This hypothesis is accurate because the system is made of: 1) the atomizing catheter, which is a long thin line filled with viscous liquid, therefore it is well approximated by a hydraulic resistance, and 2) compliances that are mainly due to bubbles and the mechanical frame. This model, according to previous experiences and testing activity is quite simple but able to describe the system, although it does not take into account non-linarites, that can be introduce by the infusion pump or inertial contributions, which are considered as negligible since the amount of mass of pulmonary surfactant line is minimal.

This model makes evident that the rising and falling time issues are mainly related to the intrinsic low pass filtering behavior of the system which is a single pole one.

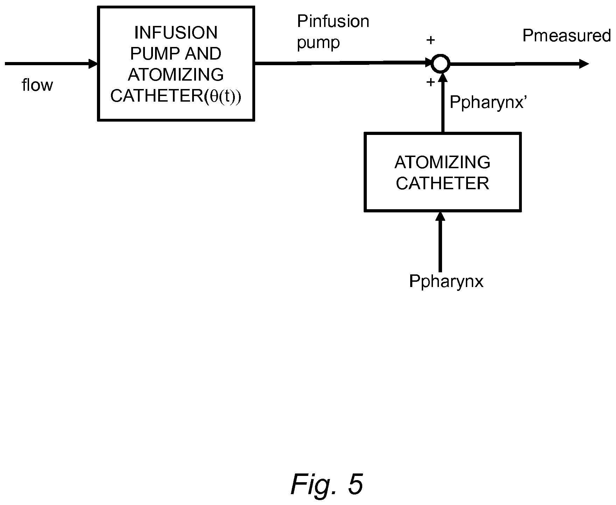

Moving from the electrical representation to a block scheme, we obtain the representation of Figure 5 which explicates that the pressure at the pharynx (Ppharynx) is not directly transduced to the pressure sensor along the medicament line, but that it is affected by the hydraulic behavior of the atomizing catheter and changed into Ppharynx'.

In this representation, $\theta$(t) vector encompasses all the characteristics of the infusion pump and atomizing catheter system and enlighten time dependency.

According to this implementation, the system is defined as in Equation 1,

$$\text{Equation 1} \qquad \theta(t)=[R(t)\ C(t)]'$$

In a real case, the mechanical characteristics of the device are changing time to time because, even if the resistance of the surfactant lumen of the catheters is very reproducible, the amount of bubbles dissolved into the surfactant may change significantly and unpredictably. For this reason, the device cannot be considered as a time-independent system. Moreover, during the delivery of the surfactant, some bubbles may flow out of the catheter lowering the actual compliance of the system.

Getting into the Laplace domain, the system can be defined by its Laplace transformed as in Equation 2 and 3:

Equation 2          *Infusion Pump and atomizing catheter*

$$= Sys1(s) = \frac{PinfusionPump}{Flow}$$

$$= \frac{R}{1 + sRC}$$

Equation 3

$$Atomizing\ catheter = Sys2(s) = \frac{Ppharynx'}{Ppharynx} = \frac{1}{1 + sRC}$$

## HOW TO REDUCE RISING AND FALLING TIME

In a first aspect of the present invention, a new approach to shorten rising and falling time is described. The aim of this approach is to make the delivery of the medicament more efficient and more coherent with the signal triggering the starting/stop of the volumetric pump connected to the medicament line, avoiding the wasting due to the flow generated by the discharging of the compliant element, Figure 3.

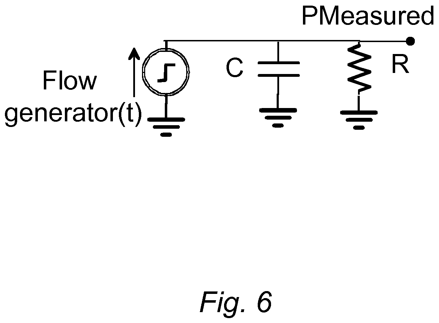

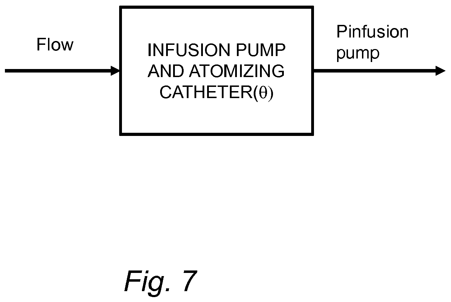

For the sake of simplicity, as the effects of the pharyngeal pressure will be neglected being much smaller than the pressure swing due to the activation of the pump, the system can be modelled as in Figure 6 and its correspondent block scheme described in Figure 7.

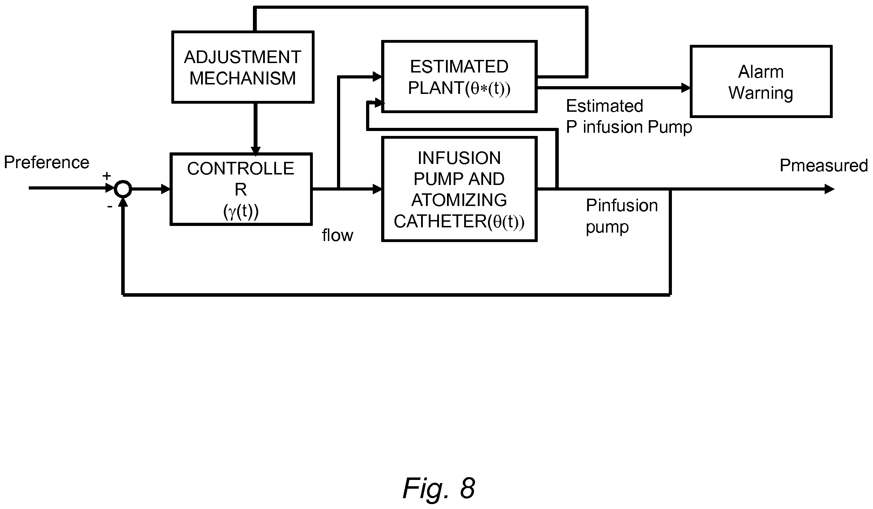

The first embodiment (Embodiment 1) is represented in Figure 8. The mechanical properties of the "INFUSION PUMP AND ATOMIZING CATHETER" block, described by θ(t), are unknown. The parameters θ(t) of the model are continuously identified and associated to an "ESTIMATED PLANT", described by θ(t)*. Once the estimated resistance (R(t)*) and compliance(C(t)*) of the system are known, a decisional tree is implemented according to their values. If they are out of an acceptable range, an "ALARM WARNING" block will warn the operator about malfunctioning of the system, otherwise the delivery will start (or will go on). The identified values of R(t)* and C(t)* are used even to select the best approach for the management of the pump by affecting the CONTROLLER block. The parameters describing the CONTROLLER ,encompassed in the vector y(t), will be adjusted, thanks to an "ADJUSTMENT MECHANISM", according to the values of R(t)* and C(t)* within a predefined set of combination.

The goal of the closed loop block, CONTROLLER, is to control the motor of the infusion pump to make the pressure measured along the surfactant line more similar as possible to an ideal pressure target point selected previously and corresponding to the desired surfactant flow rate during the delivery time and zero during the hold time (i.e. during the expiratory phase).

Control theory provides several strategies to design a CONTROLLER, such as the optimal control theory, in which the parameters describing the CONTROLLER can be chosen in an infinite domain and can assume any values. Nevertheless, this approach makes impossible to predict the behaviour of the controller for any possible set of identified parameters, being them infinite. As a consequence, possible issues on the reliability and safety of the system can arise because some parameters, although solving the mathematical problem, could be not suitable to the specific mechanical system. In this invention, instead, we use an approach based on a look up table. Few working conditions for the "INFUSION PUMP AND ATOMIZING CATHETERS" were identified according to the mechanical properties of the "ESTIMATED PLANT" and then a CONTROLLER characterized by a set of predefined and tested values, one for each possible condition of the "INFUSION PUMP AND ATOMIZING CATHETERS", was used. This approach relays intrinsically on a finite number of possible working conditions, which makes possible testing each of them granting for safety and a known behaviour of the system.

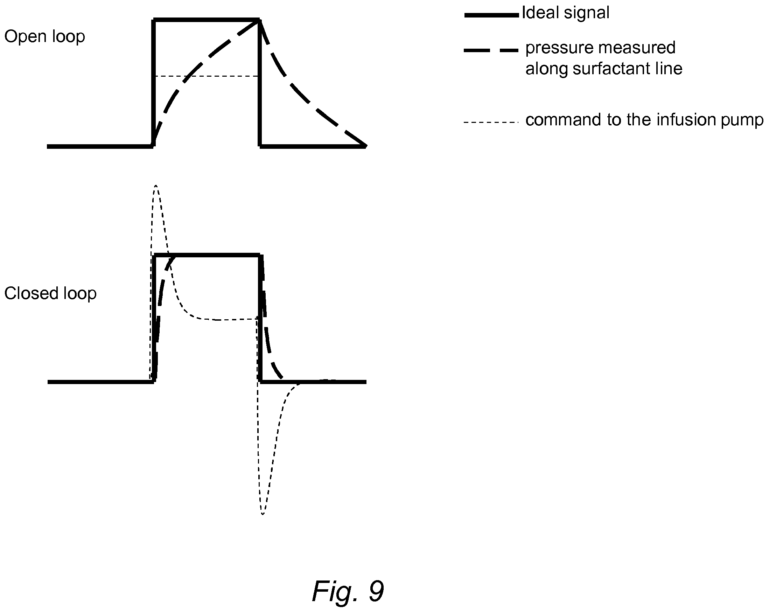

In more details, surfactant flows at a given flow rate (for instance 1.2mL/h) and it produces a certain pressure drop at the inlet of the atomizing catheter which can be measured. The pressure drop is linearly related, (since the flow is laminar such as in this application) to the flow rate by a coefficient that is the hydraulic impedance of the system. The amount of pressure drop needed to produce the desired flow is constant unless there are big changes in the physical characteristics of the system (for example, catheter occlusion). The pressure in the catheter does not reach instantly the desired level of pressure when the motor of the infusion pump is turned on because of the compliance of the system (with "compliance of the system", we mean that behaviour that, in combination with the high resistance of the atomising catheter, introduces a loss in the performances, e.g. elastic behaviour of the mechanical frame of the infusion pump and, most importantly, gas bubbles in the surfactant circuit). If the motor is controlled by an appropriate close loop control low, in case of low time constant the controller will drive the motor to rotate faster, allowing the desired pressure in the surfactant line to be reached faster. Once the target pressure has been reached, the controller will slow down the rotation of the motor as reported in Figure 9, avoiding overshooting. The pressure sensor along the surfactant line can be used to assess the actual pressure and to regulate the velocity of the motor, accordingly to a previously defined control law with the final aim to keep the flow constant and to reduce the rising and falling times.

Besides, identification block is a core feature of the system because it allows even to trigger the "ALARM AND WARNING" block thanks to:

1) Detection of the resistance in real time. Since the resistance is supposed to be constant because it is due to the dimension of the atomizing catheter, an increase may be due to an occlusion or to kinking of the catheter.
2) Estimation of the amount of bubble in the medicament line. Immediately after the circuit has been primed, the identification algorithm estimates the compliance and, if it is too high, the device can warn the operator.

IN VITRO TESTING ACTIVITY

A possible embodiment of the system that allows reaching the aim of controlling the delivery of the atomization on the basis of the identified model is reported in the following paragraphs. A short description of the device, (atomizing device) will be provided, followed by a possible implementation of each block described in Figure 8 and the performances obtained. At the end of the paragraph, general remarks on the whole system will be presented.

*Atomizing device*

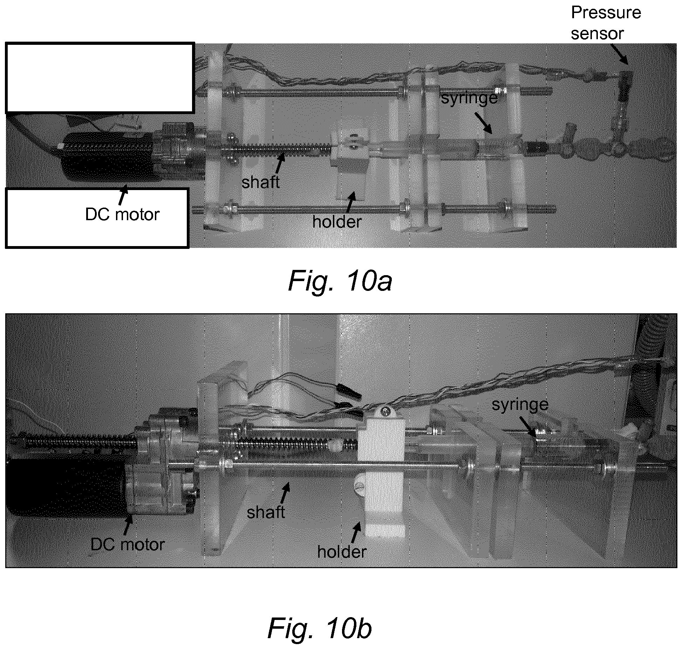

The device consists of a modified atomization device, as represented in Figure 10, made of an ad-hoc volumetric pump system (infusion pump). The system, in the present example, is made of:

1) A low cost DC motor with an encoder mounted on the rotor. The shaft of the motor is coupled with a gear to produce linear motion from the motor rotation.
2) A pressure sensor connected to the surfactant line.
3) An electronic unit comprising a) a control unit; b) a signal conditioning board for the pressure sensor; and c) the driver circuits for controlling the motor.
4) A possible embodiment of the atomizing catheter as reported in PCT/EP2014/072278.
5) A gas compressor and a pressure regulator to generate and control the atomizing airflow.
6) An *ad hoc* plastic holder, developed to rigidly anchor the piston of the syringe to the shaft of the motor. Therefore, the shaft can pull and push the syringe piston.
7) Plexiglas frame developed to align the shaft to the syringe and to make the structure robust.

In this example embodiment, the syringe is filled with Curosurf® and the output of the syringe is connected to an atomizing catheter via a low-resistance/low-compliance tube. The tip of the atomizing catheter is inserted into a test lung where it senses a pressure swing similar to the one due to breathing activity. The pressure of the surfactant line is sensed at the output of the syringe by a pressure sensor.

*System Identification*

Infusion Pump and atomizing catheter block has been described in Equation 2. Given the equation, there are several approaches for identifying the values of the parameters which may be selected on the base of the computational resources that are available and on the electronic controller unit.

A very well-known general approach is based on the Nelder-Mead Simplex Method which is able to solve any kind of minimization problem, but since our problem is linear in the parameters space, we decided to use a recursive least square algorithm (RLS).

RLS has two advantages: 1) it doesn't require high computation resources; 2) its objective function presents only a global minimum.

Recursive algorithm updated their parameter at any new given sample; therefore they are able to describe time varying models. In order to make the algorithm faster in following the variation of the parameters, we introduced also a forgetting factor.

The RLS algorithm should be able to properly identify the system even during the action of the controller, because the parameters of the controller itself rely on the state of the system and therefore they should be changes as the system changes.

*Protocol*

The system is primed with Curosurf® trying to avoid bubbles or foam. After this, a known volume of air was inserted into the circuit to change the compliance. For each condition we:

1. Started the motor in open loop (that is without being connected to the controller), and allowed it to reach the desired target flow, 1.2mL/min;
2. Stopped the motor and waited for recovery;
3. Started the motor, with the same amount of bubbles but activating the close loop controller.

The open loop measurement is equivalent to the step response of the system. It is used to estimate the parameters by using the the Nelder-Mead Simplex Method, that we considered as the gold standard.
RLS with forgetting factor was used to estimate the same parameters during the activation of the pump in closed loop.

*Results*

The parameters estimated by the RLS method and by the Nelder-Mead Simplex Method were compared in Table 1. They are express in arbitrary unit.

Table 1: Comparative table

| Bubble(ml) | Nelder-Mead Simplex Method | | RLS forgetting factor | | %error R | %error Tau | Number of repetition to converge |
|---|---|---|---|---|---|---|---|
| | R (AU) | Tau (AU) | R (AU) | Tau (AU) | | | |
| 0.1 | 0.05759 | 0.368 | 0.05708 | 0.3653 | -0,89 | -0,73 | 3 |
| 0.2 | 0.06700 | 0.6173 | 0.06721 | 0.6287 | 0,31 | 1,85 | 4 |
| 0.5 | 0.06132 | 1.432 | 0.06236 | 1.437 | 1,70 | 0,35 | 3 |
| 0.6 | 0.06570 | 1.759 | 0.06357 | 1.794 | -3,24 | 1,95 | 4 |

Remarks:

- Since the atomizing catheter is always the same, we should expect that the resistance being constant and independent from the presence and amount of bubbles. We observed a slight increase of the resistance (with both methods), likely due to some non-linearity affecting the model when high compliances values were reached.
- The resistance is estimated with a very small error, less than 4 %. The same is for the compliance that showed an error smaller than 4%.

These results suggest that the RLS method is reliable in the estimation of the model.

<u>*Controller*</u>

The control science theory deals with several strategy to optimize the control low for a dynamic system. We decided to implement the controller by means of a PID controller, whose parameters are optimized in order to make the response of the system as fast as possible and they are selected according to the amount of bubbles as reported by a look up table found empirically.

*Protocol*

The atomizing system was the one described above.
The system is primed with Curosurf® trying to avoid bubbles or foam, although we know it is really difficult to avoid bubbles at all. Then a known volume of air is inserted into the circuit to change the compliance and a sweep is performed. A sweep consists in:

4. Starting the motor in open loop, which is without controller, and to make it reaches the desired flow, 1.2mL/min;

5. Stopping the motor and waiting for recovery;

6. Starting the motor, with the same amount of bubbles, and to control it in order to achieve the pressure target as fast as possible and to keep the pressure steady.

The amount of bubbles inserted on purpose are the one listed below, they obviously represents the amount of gas that the user might unwillingly insert.

1) 0.3 ml
2) 0.5 ml
3) 0.7 ml
4) 0.9 ml

*Results and discussions*

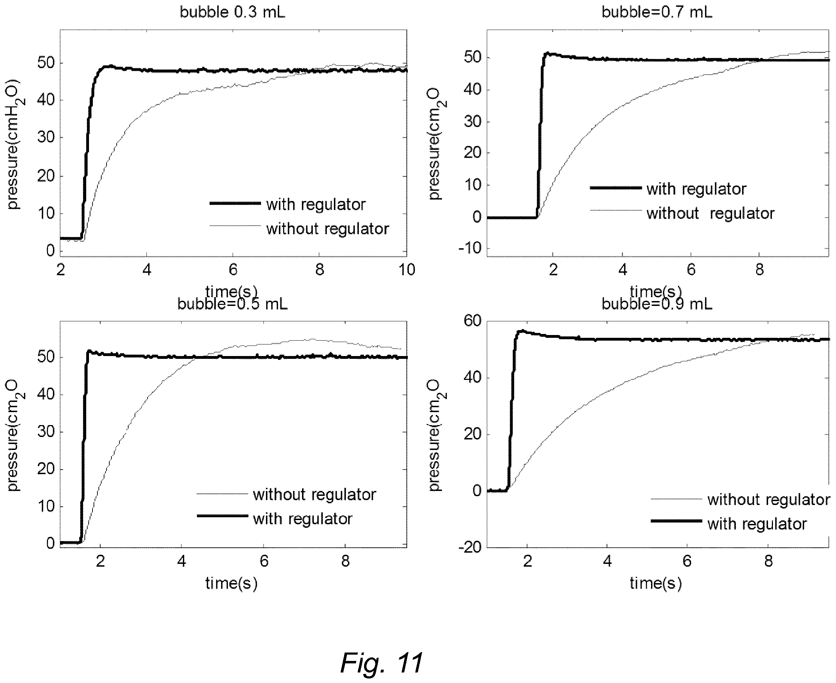

In the table below, the rising time is reported for any amount of bubbles. Rising time is defined as the time needed by the pressure to rise from 10 to 90% of the final level. Results are also represented in Figure 11.

Table 2: bubbles inserted into the syringe and a comparison between rise time with and without the regulator.

| Bubble (mL) | Rising time (without regulator) (s) | Rising time (with regulator) (s) |
| --- | --- | --- |
| 0.3 | 1.57 | 0.08 |
| 0.5 | 2.35 | 0.09 |
| 0.7 | 4.90 | 0.12 |
| 0.9 | 5.38 | 0.13 |

It is clear that, by using the controller, the time needed to establish a proper atomization is shortened up to 50 times and, moreover, the region where the surfactant flow is expected to be constant is flatter as the controller acts in order to keep the system on the target values.

*Adjustment Mechanism*

In this embodiment the adjustment mechanism is performed by creating a look up table that associates the parameters of the controller at the system identified. In this embodiment, the use of a table with a pre-defined number of states instead of a free self-adaptive system allows to pre-define all the possible values of the parameters and, therefore, determine all possible behaviour of the controller action. Limiting the controller action in a pre-determined range allows to warrant that the controller will always work in a safe way avoiding unpredictable behaviour of the controller that could arise when unexpected events of malfunctions affects the measured variables.

Table 3 reports the PID parameters as optimizes at any given amount of bubbles and the rising time in open and closed loop.

Table 3: PID parameters

| Bubble volume [ml] | Kp | Ki | Kd | Open loop [s] | Rise time - mean±std [ms] |
| --- | --- | --- | --- | --- | --- |
| 0.1 | 0.065 | 1.00E-04 | 0.4 | 0.957 | 44±6 |
| 0.2 | 0.095 | 1.00E-04 | 0.4 | 1.593 | 46±8 |
| 0.3 | 0.145 | 1.00E-04 | 0.6 | 2.216 | 49±7 |
| 0.4 | 0.135 | 2.00E-04 | 0.4 | 3.135 | 47±3 |
| 0.5 | 0.145 | 1.00E-04 | 0.44 | 4.026 | 81±2 |
| 0.6 | 0.155 | 2.00E-04 | 0.4 | 4.635 | 88±4 |

Obviously these parameters are tuned according to the specific mechanical frame, therefore they should be re-defined in case the structure of the infusion pump is changed.

*General remarks*

In conclusion, thanks to this approach three goals were achieved:

1. Reduction of the rising time of the pressure signal independently of the dimension of the surfactant lumen. This allows making the design of the catheter more independent from the time constant and therefore to make the inner lumen smaller, which, in principle, may reduce the flow of the atomizing gas.
2. Reduction of the sensitivity of the system from bubbles. This will reduce the priming time since the rise time was compatible with the application even in case of large amount of bubbles.
3. Identification of the model. This will provide useful information on the system state, for instance it provides feedback about the kinking of catheter or about the degree of obstruction of the catheter.

Moreover, this approach is very effective from a cost point of view since the regulator does not require new mechanical component nor new sensors, but it needs just a firmware (or software) adjustment.

People skilled in the art may appreciate as the non-time variant case could be considered as a simplified version of the same invention.

## HOW TO REDUCE RISING AND FALLING TIME AND TO SYNCHRONIZE THE

## DELIVERY

A second aspect of the invention is related to the detection of the breathing activity of the patient by using the pressure measure along the surfactant line. For this second aspect, the whole model of the system, as reported in Figure 4 and 5, has to be considered. The second issues related to the delivery of atomized drug into the pharyngeal region, regards the capability of the system to identify the inspiratory phase of the breathing. This invention wants to provide new concepts that allow the delivery of surfactant and the detection of the breathing phase by using only the pressure sensor present in the surfactant line and, therefore, without needing the presence of the sensing line, with advantages in terms of costs and easiness of use.

As mentioned previously and with reference to Figure 5, the actual pressure at the pharynx is not accessible, otherwise it is possible to measure a version filtered by the hydraulic impedance of the atomizing catheters, reported as Ppharynx'. Two further embodiments will be disclosed: Embodiment 2, which is based on the detection of the start of inspiration only, followed by a guess on the duration of the inspiratory phase, and Embodiment 3, which allows the recovery of the complete pharyngeal pressure signal and, therefore, the detection of both the beginning of inspiration and the beginning of the expiration.

### EMBODIMENT 2

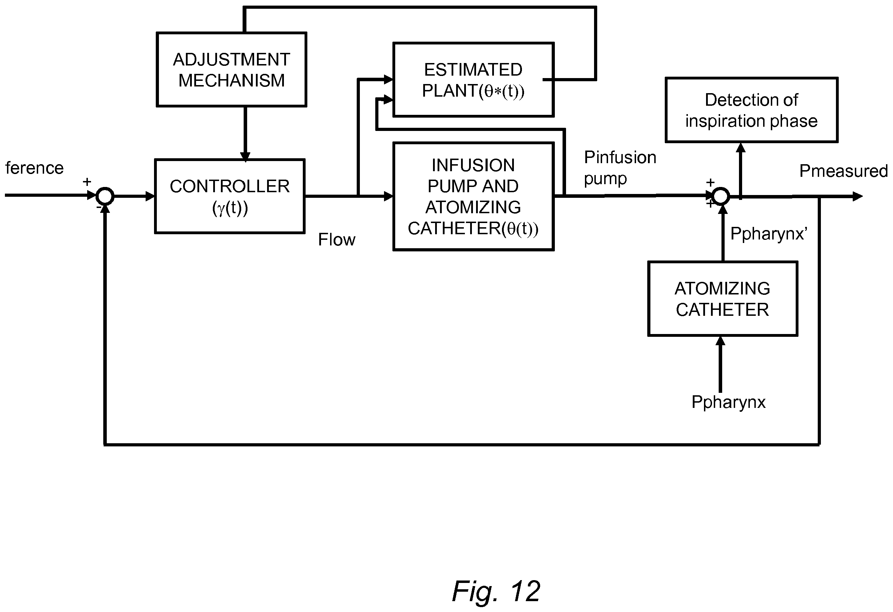

A possible approach to detect the breathing phase is the one illustrated in Figure 12:

Pmeasured is given by the sum of the contributions of the surfactant flow resulting from the intermittent activation of the infusion pump added to the pharyngeal pressure developed by the spontaneous breathing activity. As the atomizing catheter presents a high fluidodynamic resistance to the surfactant flow, the pressure generated by the infusion pump activation is usually quite bigger than the one developed by the breathing activity (in some embodiments it could be up to 10 times higher).

The embodiment 2 is based on the following consideration: during the activation of the motor, the breathing activity on the pressure signal recorded on the surfactant line can be masked by pressure drop due to the surfactant flow, conversely, when the infusion pump is not active, the breathing signal is easily detectable. Therefore, a possible solution is obtainable by detecting the beginning of the inspiration on the surfactant line (as during expiration the surfactant delivery is stopped) to start the pump activation and, as the end of expiration will be masked by the activity of the infusion pump, to stop the motor on the base of a previously estimated average inspiration time.

Expiration time can be estimated by measuring the inspiration length (Ti) and the total breathing duration (Ttot) to calculate Ti/Ttot on few spontaneous breaths of the patient done in a period in which the delivery is suspended , then it is possible to use the estimated Ti duration during the delivery phase in the following breaths in order to stop the motor at end expiration.

SIMULATION AND IN VITRO TESTING FOR EMBODIMENT 2

In order to test the performances of the invention as described in Emodiment 2, two tests were performed: 1) a mathematical simulation in which the efficiency of the medicament delivery was tested on the basis of actual pressure data recorded in the pharynx; 2) an in vitro test in which a prototype of the atomizer device has been used to deliver the medicament triggered by an emulated breathing signal generated by means of an *ad hoc* simulator.

*Simulation test*

*Protocol*

Data from 5 preterm infants receiving three different ventilatory supports were considered. Table 4 reports the characteristics of the patient population considered.

Table 4: Characteristics of the patients included in the study.

| Patient | Weight at birth [g] | Gestational age at birth [weeks+days] |
|---------|---------------------|---------------------------------------|
| #1 | 1415 | 32 |
| #2 | 1420 | 32 |
| #3 | 2340 | 32+4 |
| #4 | 1680 | 32+4 |
| #5 | 1690 | 31 |

On these patients, the following recordings were available:

1) Respiratory chest wall volume measured by Respiratory Inductance Plethysmography(RIP);
2) Pharyngeal pressure measured by a catheter-transducer system inserted into the pharynx of the patient.

The beginning and end of each inspiration were detected on the RIP signal in a semiautomatic way and these values were considered as reference for the comparisons.

The algorithm was run on the pharyngeal pressure signal. The assumption is that, during the activation of the motor the end of inspiration cannot be detected from the pharyngeal pressure signal. The algorithm works as a two finite states machine. As soon as one state terminates, the algorithm switches to the other state:

1) State one: breathing pattern parameters initialization state. The delivery of the treatment is suspended for few breaths (for example ten) and during this period end-expiration and end-inspiration data point are identified on the pharyngeal pressure and these data are used to estimate the average Ti/Ttot. Once the estimation of the breathing pattern parameters is concluded, the algorithm switches into the delivery state.

2) State two: delivery state. In this state, the algorithm detect the beginning of inspiration on the pharyngeal pressure signal and uses the estimated value of Ti obtained in state one to stop the infusion pump at end-expiration. As the values of Ti might change during the delivery, the delivery state runs for a pre-determined period (for example 50,100,150,200 or 250 seconds) after which the delivery is suspended and the system is switched back in state one to produce a new updated estimate of Ti.

*Results and discussions*

Two parameters were considered to evaluate the performances of the algorithm:

- The ratio between the time of activation of the infusion pump determined by the algorithm during inspiration versus the time of the actual duration of the inspiration of the patient;
- The ratio between the time of activation of the infusion time during the expiration of the patient versus the total duration of the breath The results are reported in Table 5 (performances of the algorithm).

Table 5: performances of the algorithm

| Initialization of breathing pattern parameters every X seconds | Percentage of surfactant delivered during an actual inspiration | Percentage of surfactant delivered during expiration (wasted) |
|---|---|---|
| X=50 | 75.0±6.2 | 5.8±2.5 |
| X=100 | 74.7±6.6 | 5.9±2.6 |

(continued)

| Initialization of breathing pattern parameters every X seconds | Percentage of surfactant delivered during an actual inspiration | Percentage of surfactant delivered during expiration (wasted) |
| --- | --- | --- |
| X=150 | 73.8±8.2 | 5.9±2.7 |
| X=200 | 73.4±9.4 | 6.0±2.7 |
| X=250 | 72.0±9.5 | 6.0±2.6 |

Table 5 shows that the treatment was delivered incorrectly only for the 6% of the total duration of the breaths and that it was properly delivered for more than 70% (in average) of the duration of the inspirations even in the worst case of X equals to 250 s.

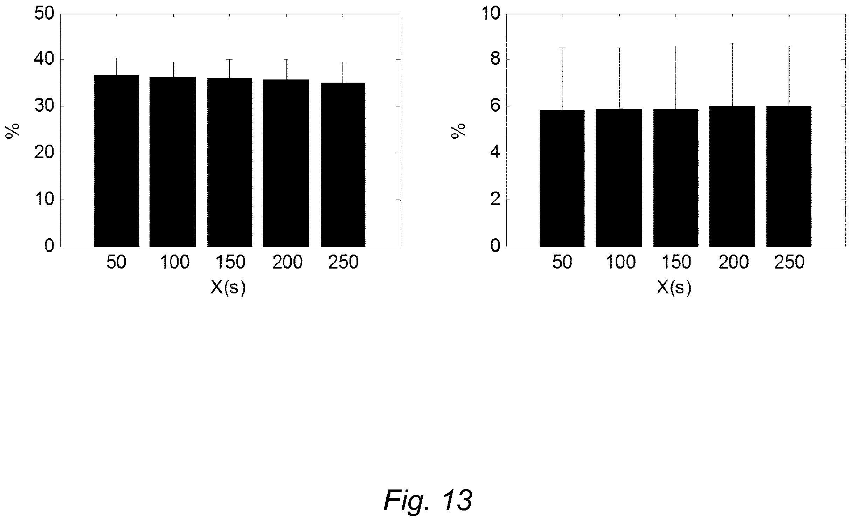

The data reported seems to state that the values of Ti estimated in the state one is quite stable with time (Figure 13). Statistical analysis was performed to clarify this aspect by one-way ANOVA test. Between the population no statistical difference was found ($p<0.0001$) as expected. Also surfactant delivery during expiration didn't show a dependence on the interval between initialization phase ($p<0.0001$), at least if it ranges from 50 to 250 seconds.

*In vitro testing activity*

*Set up and protocol*

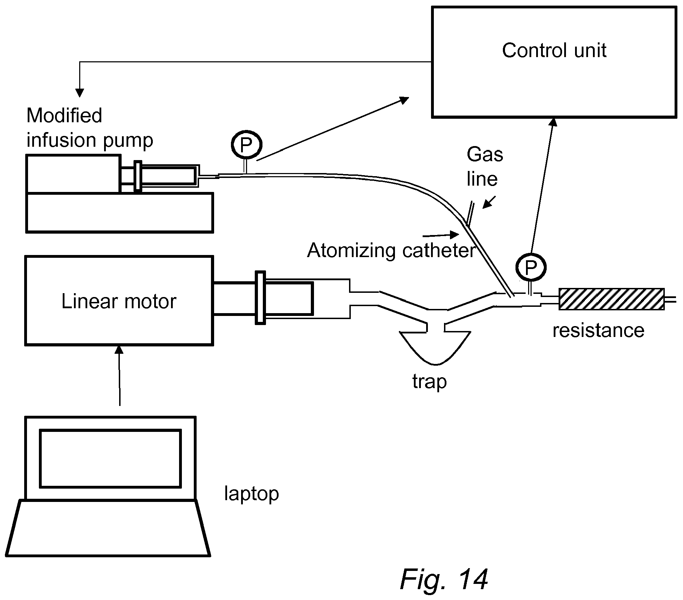

The bench test system (Figure 14) consisted of a modified atomizing device and a pressure generator able to reproduce the actual pressure signal at the pharynx. The pressure generator produced a pressure signal equals to the actual pharyngeal pressure measured on a preterm infant in a chamber. The pressure in that chamber is sensed at the same time by the atomizing catheter and by a second pressure sensor used as gold standard for further analysis.

In order to test the capabilities of the breathing detection algorithm, the possible embodiment of the atomized device described above was used. The pressure generator consisted of a servo controlled linear motor whose shaft is connected to the piston of a syringe; the motion of the motor produces a flow that is linearly related to its velocity, the flow is then producing a pressure, which is our aim, by adding an hydraulic resistance in series to the line. The pressure generated is equal to the flow times the resistance. The trap represented in Figure 14 collects the atomized liquid preventing the medicament from entering in contact with the motor/syringe embodiment.

Once the infusion pump is loaded with surfactant, the synchronisation algorithm is started. The beginning of the inspiration is detected on the pressure signal, while the beginning of expiration is based on the Ti/tot ratio estimated during the initialization state, as described above. Since simulation tests assessed that the interval between one initialization state and the following one is not a very sensitive parameter, in this experiment it has been set to 250 seconds.

*Results and discussions*

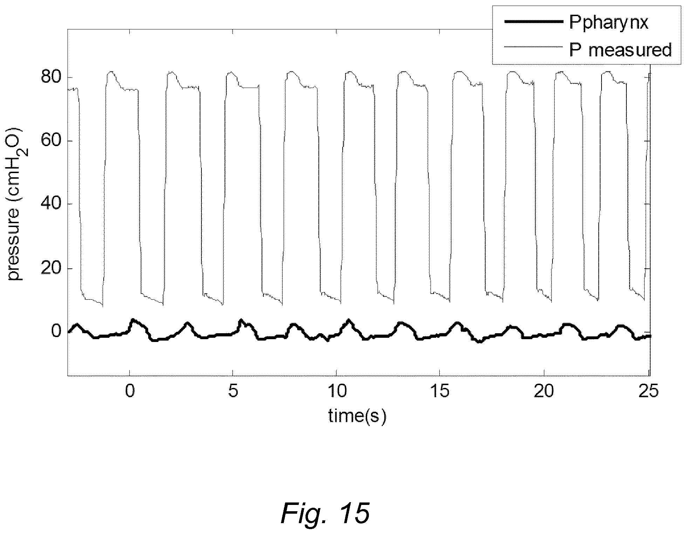

Figure 15 shows qualitative results. It can be noted that:

1) The synchronization between the activation of the motor and the beginning of the inspiration is quite responsive;
2) The system shows a very fast rising and falling time (approximatively 0.05s);
3) The system shows very reproducible pressure swings into the surfactant line, suggesting a quite fine control on the surfactant delivered.

Even if this approach is able to provide an effective solution for many applications there is still a possible limitation due to a very variable breathing pattern of the baby which may occur spontaneously or as a consequence of the treatment, that is as the medicament reaches the lung and the healing process starts. In this case the possible errors in start and stop of the delivery might become more relevant. In fact, since the end of expiration of each single breath is not actually detected but it is estimated on previous breathings, there could be relevant differences between the estimated value and the actual one in case of very irregular and time varying breathing pattern, leading to surfactant waste. Embodiment 3 discloses a solution that allows detecting both end inspiration and end expiration.

*EMBODIMENT 3: reconstruction of the whole breathing signal*

Embodiment 3 provides a solution to the problem of delivery the medicament in phase with the breathing pattern of the patient by means of a pump means which can generate a flow of medicament towards the lung of the patient in a prompt way thanks to the approach described in previous Embodiment. Embodiment 3 differs from Embodiment 2 because it provides a way to fully reconstruct the breathing activity of the patient.

This aim can be obtained at least by means of two approached as detailed below.

*EMBODIMENT 3 - APPROACH 1*

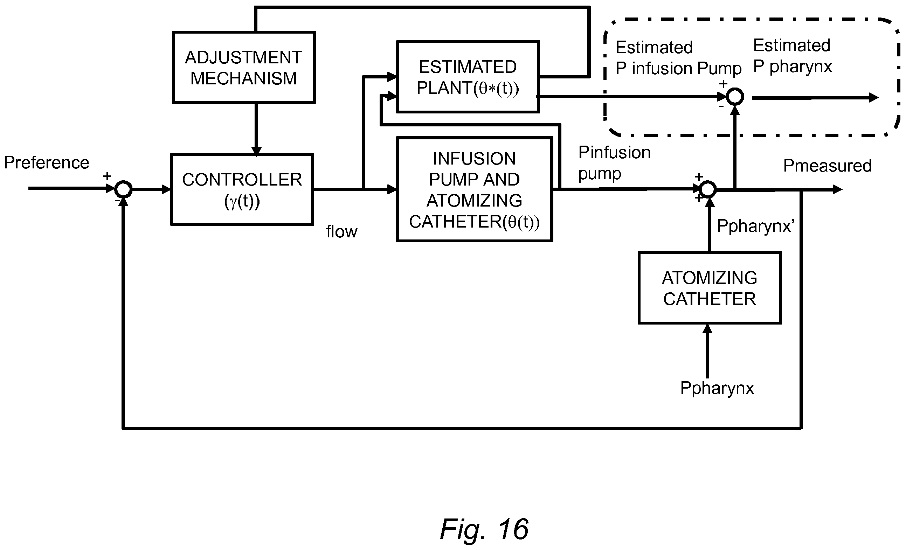

The complete working scheme of Embodiment 3- APPROACH 1 is reported in Figure 16, where the differences compared to Embodiment 2 are enclosed within a dashed line.

The controller action is acting to make the response of the system as similar as possible to the reference pressure, Preference, that is a step-like signal equals to the flow times the hydraulic resistance of the catheter.

Since the measured pressure, Pmeasured, contains also the contribution of the breathing activity, the controller action tries to compensate even for that signal, thus the controller output will contain informative content about Ppharynx'. If the infusion pump and atomizing catheter block and the estimated plant block are fed with the same signal, i.e. the output of the controller, the estimated P infusion Pump and Pmeasured will be different. This is because the output of the estimated system doesn't include the breathing activity but just the response of a fist order system to the controller action. Then, by subtracting the estimated signal to the actual measured pressure, we can obtain the respiratory signal.

SIMULATION AND TESTFOR EMBODIMENT 2

Simulations were run to test this embodiment. The following assumptions were considered:

1) the simulation requires to describe both the actual delivery system made of "infusion pump and the atomizing catheter", as reported in Figure 16, and the system that is estimated from the recorded pressure and flow, "estimated plant" as reported in Figure 16, whose parameters are identified on line continuously. As found in vitro testing of embodiment 2, the identification process introduce an average error on the estimation of the parameters that is around 5%, therefore an error of 5% was added to the parameters of the "estimate plant", respect to the parameter of .the actual "infusion pump and the atomizing catheter".

2) "Controller", as reported in Figure 16, has been chosen from PID family and it has been design on order to reach a rising and falling time of 50 ms as obtained in the in vitro testing of embodiment 2.

3) Preferably, Figure 16 represents the ideal pressure signal recorder on the surfactant line, in case the system had no compliance at all. It is a step signal. We run two simulations considering two different amount of bubbles.

The value used for the simulations are reported in the table below:

Table 6

|  | Simulation 1 | Simulation 2 |
|---|---|---|
| Resistance | 218*-109 Pa s/m$^3$ = resistance of a catheter 18cm long and with an inner diameter of 0.8 mm filled of surfactant | |
| Medicament flow | F=20*10^-9 (m$^3$/s) = flow equals to 1.2 ml/min, that is the flow needed to complete the delivery in less than 15 minutes. | |
| Compliance | C=0.9869*10-12 m$^3$ / Pa =compliance of a bubble 0.1 mL, that is a possible amount of bubble that can be inserted unwillingly | C=2*0.9869*10-12 m$^3$ / Pa =compliance of a bubble 0.2 mL, that is a possible amount of bubble that can be inserted unwillingly |

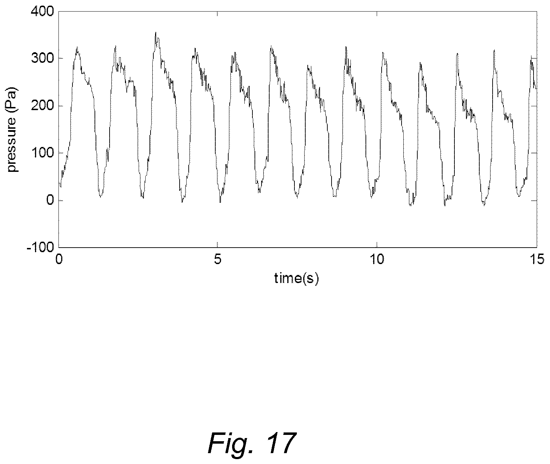

Ppharynx used in both simulations is the pharyngeal pressure measured in a term piglet breathing spontaneously under CPAP. The piglet weights 1 kg. This could be considered a good reference for the baby breathing pattern, Figure 17.

*Results*

*Rising and falling time*

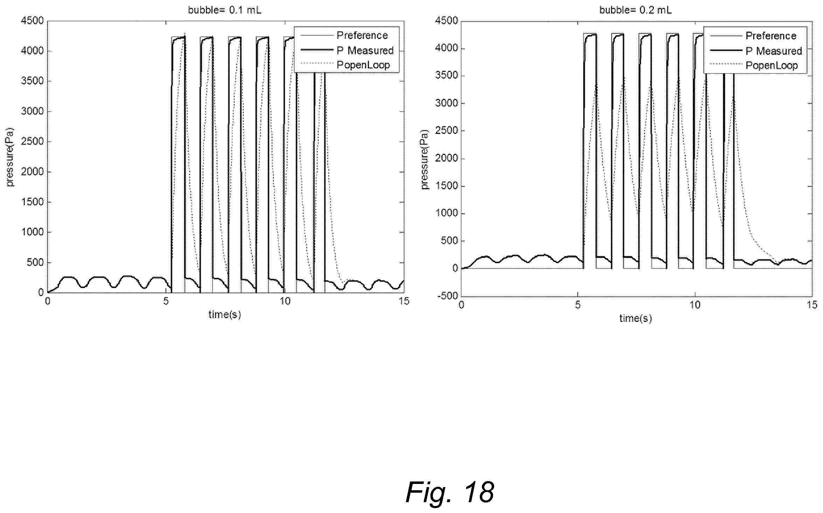

Figure 18 shows the rising and falling time obtained with two different amounts of air bubbles. In the same figure, the open loop response of the system is reported too. It is interesting to notice that with a total amount of gas bubbles equals to 0.1 mL, the amount of surfactant delivered during expiration (that is wasted) is comparable to that delivered during inspiration. In case of more bubbles, 0.2 mL, the system cannot work properly and the surfactant is delivered continuously, even during expiration.

*Detection of pharyngeal pressure*

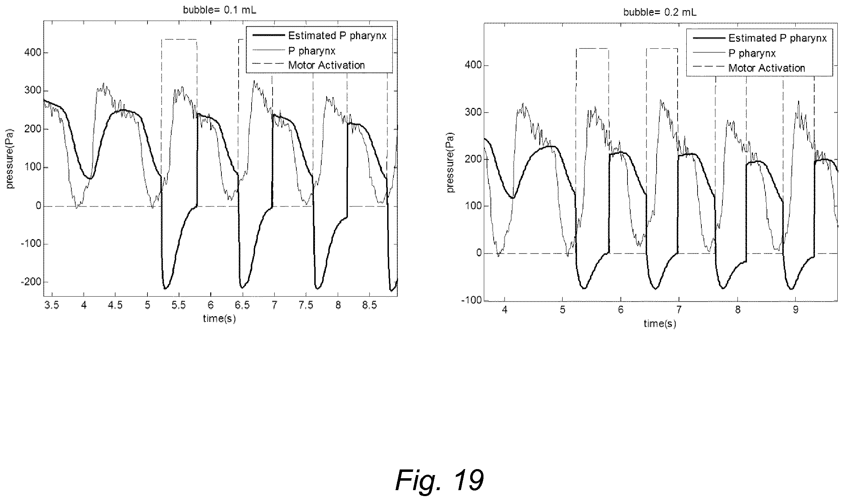

In Figure 19 the estimated pharynx pressure signal is reported and compared to the actual one. Although the strong low pass effect and the abrupt changes as the motor was turned on and off, it is still possible to identify the breathing phase. APPROACH 2

*EMBODIMENT 3 - APPROACH 2*

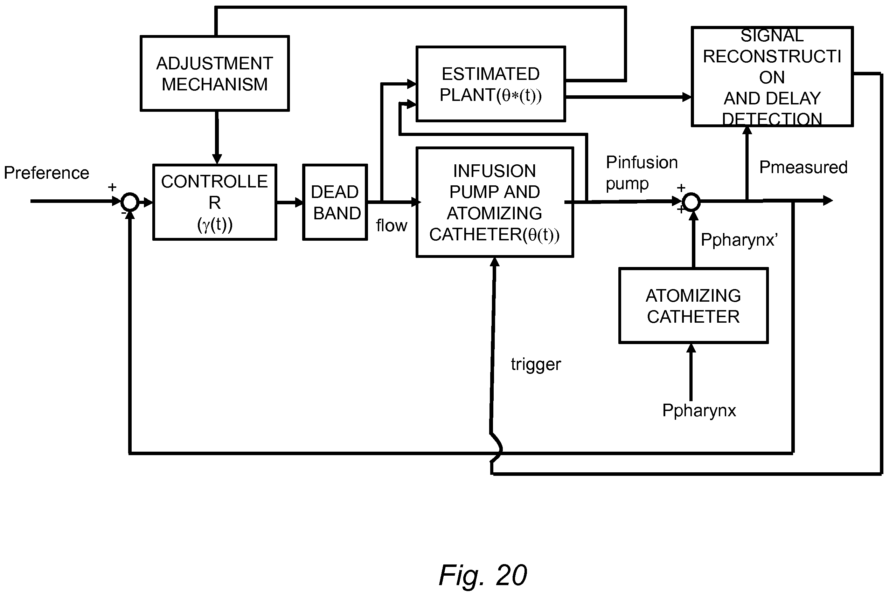

The last embodiment, embodiment 4, presents an approach that is able to totally reconstruct the signal and even to compensate for the delay on the surfactant line. If the time constant of the system is particularly high, that means the delay between the actual pharyngeal pressure, Ppharynx, and the recorded one, Ppharynx', is too high the afore mentioned concepts could present some limitations that are overcome by this approach Figure 20.

Scheme of Embodiment 2-APPROACH2 differs from Embodiment 2-APPROACH1, Figure 12, because of two blocks:

    1) Dead band
    2) Signal reconstruction and delay detection.

The purpose of these blocks is discussed in detail here below:

*Dead Band*

In the previous concepts, the controller action has two aims: to compensate for the time constant and to compensate for the effect of the breathing activity that is removed from the signal measured, Pmeasured.

The insertion of the dead band block makes the controller able to compensate only for the time constant effects and not for the breathing activity. This result is obtained by setting the resolution used by the controller to change the flow bigger than the amplitude of the breathing signal, which is several times smaller than the activation signal. In other words, the changes in the measured pressure due to the breathing activity, are under threshold to be considered by the controller and does not results in actions on the infusion pump.

As a finale result, which is the aim in inserting the dead band, Pmeasured contains the breathing activity both when the motor is activated under the controller action and both when the motor is turned off.

*Signal reconstruction and detection of the phase delay*

*Signal reconstruction*

Thanks to dead band block, the breathing signal is always detectable on Pmeasured except during the transition of the motor from on to off and vice versa. Since the pressure step in these phases is known, it is possible to fully reconstruct the signal by removing it from PReference. This will result in some blanking time limited to rising and falling time but, if the controller works properly, they will be limited to few milliseconds.

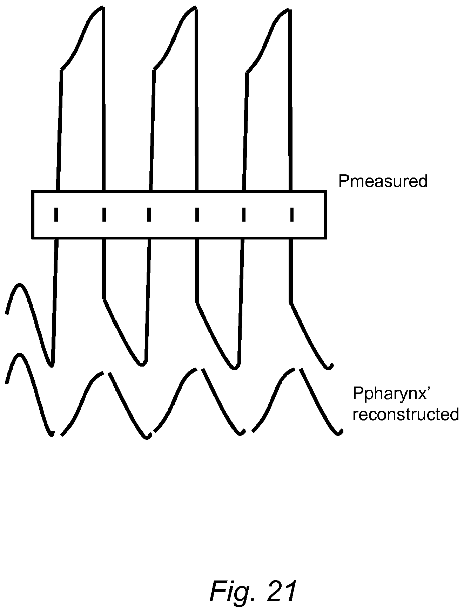

Figure 21 shows this concept: top) Pmeasured contains breathing activity, bottom) the pharyngeal pressure reconstructed by removing the activation signal due to the motor activation.

*Detection of delay in the sensing signal*

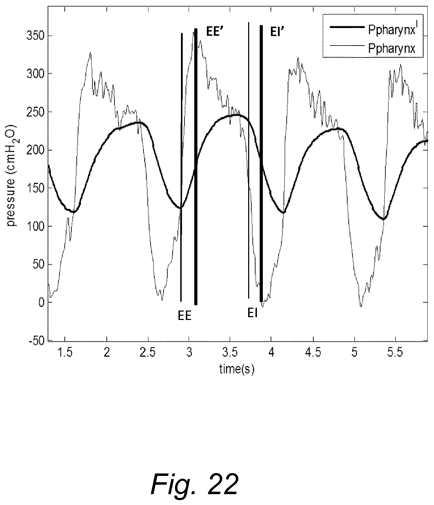

Although dead band allows reconstructing the breathing signal, there is still the issue associated to the delay added by the mechanical characteristics of the catheter (Figure 22).

The actual end of expiration, EE, is delayed compared to the measured one, EE', that means the delivery of the drug would not be in phase with the actual breathing but it would be delayed of a time that depends on the time constant of the system and the breathing frequencies and can be calculated by Equation 4, where Sys 2 is the block representing the atomizing catheter as in Equation 3 and $T_{breathing}$ is the inverse of the respiratory rate.

$$\text{Equation 4} \quad T_{delay} = T_{breathing} * \frac{\measuredangle Sys2\left(2\pi \frac{1}{T_{breathing}}\right)}{2\pi}$$

*Trigger signal for embodiment 5*

In order to work properly, the atomising system should be promptly triggered at the beginning of inspiration. To reach this aim, all the elements described above have to be combined together:

1) The reconstructed signal is delayed of a time, $T_{delay}$, that could be estimated by means of the estimated parameters of the system.
2) As shown above, with reference to Embodiment 2-APPROACH 1, given the pharyngeal pressure signal it is possible to predict with a good accuracy the duration of the next inspiration and the next expiration.

Therefore, once the end inspiration on the reconstructed signal has been detected, the next activation of the motor, $T_{trigge}$r, Equation 5, would happen after a time equals to the mean duration of the expiration, $T_{expiration'}$, corrected by the delay introduced by the surfactant line, $T_{delay}$ as estimated in Equation 4.

$$\text{Equation 5} \qquad T_{trigger} = T_{expiration'} - T_{delay}$$

*In vitro testing activity*

The aim of the in vitro testing activity is to prove the feasibility of the approach that can be inferred by the capability of the system to reconstruct the pharyngeal pressure.

*Set up*

The set up was the same reported for Embodiment 2. The amount of bubble added is 0.2 mL.

*Results*

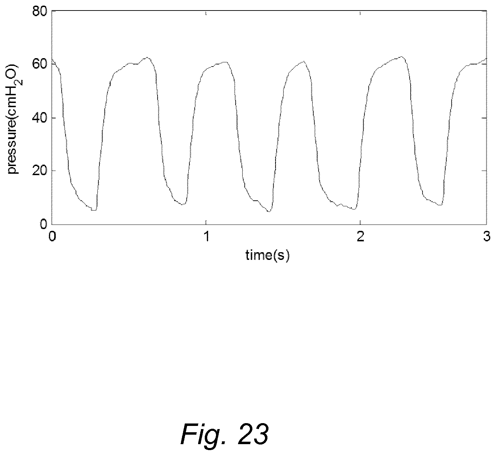

Figure 23 shows the pressure measured along the surfactant line during start and stop of the motor. In this embodiment, the controller has been tuned to minimize extra-shootings in the pressure signal even though this has been slightly increased the rising and falling times. Breathing activity is still difficult to detect because it is partially masked by the turning on and off of the motor.

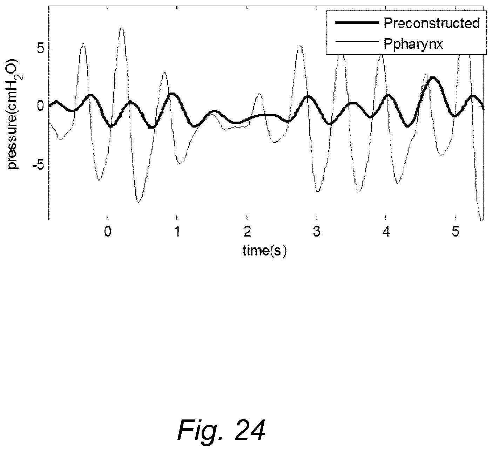

Figure 24 shows the reconstructed signal compared to the actual pressure. They present a similar trend and although the reconstructed signal has a slightly different shape compared to the actual one, end inspirations and expirations are well detectable.

Advantageously, the system of the invention is applied to pre-term neonates who are spontaneously breathing, and preferably to extremely low birth weight (ELBW), very-low-birth-weight (VLBW), and low-birth weight (LBW) neonates of 24-35 weeks gestational age, showing early signs of respiratory distress syndrome as indicated either by clinical signs and/or supplemental oxygen demand (fraction of inspired oxygen ($FiO_2$) > 30%).

As non-invasive respiratory support, in a preferred embodiment, nasal Continuous Positive Airway Pressure (nCPAP) could be applied to said neonates, according to procedures known to the person skilled in the art. Preferably a nasal mask or nasal prongs are utilised. Any nasal mask commercially available may be used, for example those provided by The CPAP Store LLC, and the CPAP Company.

Nasal CPAP is typically applied at a pressure comprised between 1 and 12 cm water, preferably 2 and 8 cm water, although the pressure can vary depending on the neonate age and the pulmonary condition.

In another preferred embodiment, nasal intermittent positive-pressure ventilation (NIPPV) could be applied.

Other non-invasive ventilation procedures such as High Heated Humidified Flow Nasal Cannula (HHHFNC) and bi-level positive airway pressure (BiPAP) could alternatively be applied to the neonates.

**Claims**

**1.** A system for delivering a liquid pulmonary surfactant to spontaneously breathing patients, comprising:

- i) a catheter adapted to reach the pharyngeal region of the patient, the catheter including at least a first channel being adapted to convey in the patient's pharyngeal region a flow of liquid pulmonary surfactant,
- ii) first pump means connected to a first end of the at least first channel, adapted to create a pressure which pushes the column of liquid pulmonary surfactant towards the second end of the at least first channel;

- iii) breathing detecting means, for measuring a value indicative of whether the patient is in an inspiration or in an expiration phase;
- iv) pressure detecting means connected to the first channel for measuring a value indicative of the pressure of the liquid pulmonary surfactant;
- v) a microprocessor configured to selectively activate the first pump means according to signals received from the breathing detecting means and the pressure detecting means, so that the first pump means are activated only during inspiration phase and the flow produced by the first pump means is adapted to counterbalance the delay induced by the hydraulic impedance of the system; **characterized in that** the first pump means includes a volumetric pump and the hydraulic impedance is estimated according to the measured value of the pressure in the first channel and the volume delivered by the pump;
wherein adapting the flow produced by the first pump means includes adapting the flow according to a function having a plurality of predetermined sets of coefficients, each set of coefficients being associated to a range of values of the estimated hydraulic impedance.

2. The system of claim 1 wherein adapting the flow produced by the first pump means includes increasing the initial flow of the first pump means until the pressure measured by the pressure detecting means reaches a predetermined value.

3. The system of claim 1 wherein the predetermined sets of coefficients are stored in a lookup table accessible by the microprocessor.

4. The system of any preceding claim, wherein breathing detecting means includes a pressure sensor, for measuring a value indicative of the pressure in the patient pharyngeal cavity, such value being used to determine whether the patient is in an inspiration or in an expiration phase.

5. The system of claim 4 wherein the determination of whether the patient is in an inspiration or in an expiration phase is done by detecting the start of the inspiration phase and calculating the end of the inspiration phase according to predetermined values indicative of the duration of the inspiration phase.

6. The system of claim 4 or 5, wherein the pressure sensor coincides with the pressure detecting means connected to the first channel.

7. The system of any preceding claim, wherein the catheter includes at least a dedicated gas channel adapted to convey in the patient's pharyngeal region a pressurized flow of gas, the system further comprising:

- gas pump means connected to a first end of the gas channel, adapted to create the flow of pressurized gas; so that when the column of liquid pulmonary surfactant and the pressurized gas meet in the pharyngeal cavity, the liquid column is broken into a plurality of particles causing the atomized pulmonary surfactant to be delivered into the patient's lungs.

**Patentansprüche**

1. System zum Liefern eines flüssigen pulmonalen oberflächenaktiven Stoffs an spontan atmende Patienten, umfassend:

- i) einen Katheter, welcher dazu eingerichtet ist, die Rachenregion des Patienten zu erreichen, wobei der Katheter wenigstens einen ersten Kanal umfasst, welcher dazu eingerichtet ist, einen Strom eines flüssigen pulmonalen oberflächenaktiven Stoffs in den Rachenbereich des Patienten zu befördern,
- ii) erste Pumpenmittel, welche mit einem ersten Ende des wenigstens ersten Kanals verbunden sind, welche dazu eingerichtet sind, einen Druck zu erzeugen, welcher die Säule des flüssigen pulmonalen oberflächenaktiven Stoffs in Richtung des zweiten Endes des wenigstens ersten Kanals drückt;
- iii) Atmung-Detektion-Mittel zum Messen eines Werts, welcher anzeigt, ob der Patient in einer Einatmung- oder in einer Ausatmung-Phase ist;
- iv) Druck-Detektion-Mittel, welche mit dem ersten Kanal zum Messen eines Werts verbunden sind, welcher den Druck des flüssigen pulmonalen oberflächenaktiven Stoffs anzeigt;
- v) einen Mikroprozessor, welcher dazu eingerichtet ist, selektiv die ersten Pumpenmittel gemäß Signalen zu aktivieren, welche von den Atmung-Detektion-Mitteln und den Druck-Detektion-Mitteln empfangen werden, so dass die ersten Pumpenmittel nur während einer Einatmung-Phase aktiviert werden und der Strom, welcher durch die ersten Pumpenmittel erzeugt wird, dazu eingerichtet ist, die Verzögerung auszugleichen, welche durch die hydraulische Impedanz des Systems induziert wird;

**dadurch gekennzeichnet, dass** die ersten Pumpenmittel eine volumetrische Pumpe umfassen und die hydraulische Impedanz gemäß dem gemessenen Wert des Drucks in dem ersten Kanal und dem Volumen geschätzt wird, welches durch die Pumpe geliefert wird; wobei das Anpassen des Stroms, welcher durch die ersten Pumpenmittel erzeugt wird, ein Anpassen des Stroms gemäß einer Funktion umfasst, welche eine Mehrzahl vorbestimmter Sätze von Koeffizienten aufweist, wobei jeder Satz von Koeffizienten einem Bereich von Werten der geschätzten hydraulischen Impedanz zugeordnet ist.

2. System nach Anspruch 1, wobei das Anpassen des Stroms, welcher durch die ersten Pumpenmittel erzeugt wird, ein Steigern des initialen Stroms der ersten Pumpenmittel bis der Druck, welcher durch die Druck-Detektion-Mittel gemessen wird, einen vorbestimmten Wert erreicht umfasst.

3. System nach Anspruch 1, wobei die vorbestimmten Sätze von Koeffizienten in einer LookUp-Tabelle gespeichert sind, auf welche durch den Mikroprozessor zugegriffen werden kann.

4. System nach einem vorhergehenden Anspruch, wobei die Atmung-Detektion-Mittel einen Druck-Sensor umfassen, um einen Wert zu messen, welcher den Druck in dem Patienten-Rachen-Hohlraum anzeigt, wobei ein derartiger Wert verwendet wird, um zu bestimmen, ob der Patient in einer Einatmung- oder in einer Ausatmung-Phase ist.

5. System nach Anspruch 4, wobei die Bestimmung, ob der Patient in einer Einatmung- oder in einer Ausatmung-Phase ist, durch Detektieren des Beginns der Einatmung-Phase und durch Berechnen des Endes der Einatmung-Phase gemäß vorbestimmten Werten erfolgt, welche die Dauer der Einatmung-Phase anzeigen.

6. System nach Anspruch 4 oder 5, wobei der Druck-Sensor mit den Druck-Detektion-Mitteln zusammenfällt, welche mit dem ersten Kanal verbunden sind.

7. System nach einem vorhergehenden Anspruch, wobei der Katheter wenigstens einen bestimmten Gaskanal umfasst, welcher dazu eingerichtet ist, einen unter Druck versetzten Strom von Gas in den Rachenbereich des Patienten zu befördern, wobei das System ferner umfasst:

- Gas-Pumpenmittel, welche mit einem ersten Ende des Gaskanals verbunden sind, welche dazu eingerichtet sind, den Strom von unter Druck versetztem Gas zu erzeugen;
so dass, wenn die Säule des flüssigen pulmonalen oberflächenaktiven Stoffs und das unter Druck versetzte Gas sich in dem Rachen-Hohlraum treffen, die Flüssigkeitssäule in eine Mehrzahl von Partikeln aufgebrochen wird, wobei die Zerstäubung von pulmonalem oberflächenaktiven Stoff hervorgerufen wird, welcher in die Lunge des Patienten zu liefern ist.

## Revendications

1. Système d'administration d'un tensioactif pulmonaire liquide pour patients es respirant spontanément, comprenant :

- i) un cathéter adapté pour atteindre la région pharyngée du·de la patient·e, le cathéter comprenant au moins un premier canal adapté pour transporter dans la région pharyngée du·de la patient e un flux de tensioactif pulmonaire liquide,
- ii) un premier moyen pompe relié à une première extrémité du au moins premier canal, adapté pour créer une pression qui pousse la colonne de tensioactif pulmonaire liquide vers la seconde extrémité du au moins premier canal ;
- iii) un moyen de détection de respiration, pour mesurer une valeur indicatrice du fait que le·la patiente·e se trouve en phase d'inspiration ou d'expiration ;
- iv) un moyen de détection de pression relié au premier canal pour mesurer une valeur indicatrice de la pression du tensioactif pulmonaire liquide ;
- v) un microprocesseur configuré pour activer sélectivement le premier moyen pompe selon les signaux reçus du moyen de détection de respiration et du moyen de détection de pression, de sorte que le premier moyen pompe est activé uniquement durant la phase d'inspiration et le flux produit par le premier moyen pompe est adapté pour contrebalancer le délai induit par l'impédance hydraulique du système ;

**caractérisé en ce que** le premier moyen pompe comprend une pompe volumétrique et que l'impédance hydraulique est estimée selon la valeur mesurée de la pression dans le premier canal et le volume administré par la pompe ;

l'adaptation du flux produit par le premier moyen pompe comprenant l'adaptation du flux selon une fonction ayant une pluralité d'ensembles prédéterminés de coefficients, chaque ensemble de coefficients étant associé à une plage de valeurs de l'impédance hydraulique estimée.

2. Système selon la revendication 1, l'adaptation du flux produit par le premier moyen pompe comprenant l'augmentation du flux initial du premier moyen pompe jusqu'à ce que la pression mesurée par le moyen de détection de pression atteint une valeur prédéterminée.

3. Système selon la revendication 1, les ensembles prédéterminés de coefficients étant stockés dans un tableau à consulter accessible par le microprocesseur.

4. Système selon l'une quelconque des revendications précédentes, le moyen de détection de respiration comprenant un capteur de pression, pour mesurer une valeur indicatrice de la pression dans la cavité pharyngée du·de la patient e, une telle valeur étant utilisée pour déterminer si le·la patient e se trouve dans une phase d'inspiration ou d'expiration.

5. Système selon la revendication 4, la détermination du fait que le·la patient e se trouve dans une phase d'inspiration ou d'expiration étant effectuée par la détection du début de la phase d'inspiration et le calcul de la fin de la phase d'inspiration en fonction des valeurs prédéterminées indicatrices de la durée de la phase d'inspiration.

6. Système selon la revendication 4 ou 5, le capteur de pression coïncidant avec le moyen de détection de pression relié au premier canal.

7. Système selon l'une quelconque des revendications précédentes, le cathéter comprenant au moins un canal de gaz dédié adapté pour transporter dans la région pharyngée du·de la patient·e un flux sous pression de gaz, le système comprenant en outre :

- un moyen pompe de gaz relié à une première extrémité du canal de gaz, adapté pour créer le flux de gaz sous pression ;

de sorte que lorsque la colonne du tensioactif pulmonaire liquide et le gaz sous pression se rencontrent dans la cavité pharyngée, la colonne de liquide est brisée en une pluralité de particules amenant l'administration du tensioactif pulmonaire atomisé au niveau des poumons du·de la patient e.

EP 3 285 645 B1

*Prior Art*

*Fig. 1*

EP 3 285 645 B1

*Fig. 2a*                                                              *Fig. 2b*

*Fig. 3*

EP 3 285 645 B1

PMeasured

R(t)

Ppharynx(t)

C(t)

Flow generator(t)

Fig. 4

*Fig. 5*

EP 3 285 645 B1

*Fig. 6*

EP 3 285 645 B1

Flow → **INFUSION PUMP AND ATOMIZING CATHETER(θ)** → Pinfusion pump

*Fig. 7*

*Fig. 8*

Open loop

Closed loop

Ideal signal

pressure measured
along surfactant line

command to the infusion pump

*Fig. 9*

Fig. 10a

Fig. 10b

*Fig. 11*

EP 3 285 645 B1

*Fig. 12*

EP 3 285 645 B1

*Fig. 13*

Fig. 14

*Fig. 15*

Fig. 16

EP 3 285 645 B1

Fig. 17

*Fig. 18*

*Fig. 19*

Fig. 20

Fig. 21

*Fig. 22*

*Fig. 23*

*Fig. 24*

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2013160129 A
- EP 2014072278 W
- WO 9532992 A
- WO 8906657 A
- WO 9222315 A
- WO 0047623 A
- WO 2010139442 A

**Non-patent literature cited in the description**

- **WAGNER MH ; AMTHAUER H ; SONNTAG J ; DRENK F ; EICHSTÄDT HW ; OBLADEN M.** Endotracheal surfactant atomization: an alternative to bolus instillation. *Crit Care Med,* 2000, vol. 28 (7), 2540